Europäisches Patentamt

European Patent Office

Office européen des brevets

(11) Publication number: **0 277 563**
**A1**

# EUROPEAN PATENT APPLICATION

(21) Application number: 88100923.7

(22) Date of filing: 22.01.88

(51) Int. Cl.⁴: **C12N 15/00** , C12N 9/90 , C07H 21/04

The applicant has filed a statement in accordance with Rule 28 (4) EPC (issue of a sample only to an expert). Accession number(s) of the deposit(s): FERM BP-1595 and IFO 14563.

(30) Priority: 28.01.87 PCT/JP87/00058

(43) Date of publication of application:
10.08.88 Bulletin 88/32

(84) Designated Contracting States:
AT BE CH DE ES FR GB GR IT LI LU NL SE

(71) Applicant: Takeda Chemical Industries, Ltd.
27, Doshomachi 2-chome Higashi-ku
Osaka-shi Osaka, 541(JP)

(72) Inventor: Yamauchi, Kiyochi
22-6, Heiwa 2-chome
Shizuoka-shi Shizuoka 420(JP)
Inventor: Horiuchi, Ryuya
1060-1, Oaza-komuro Kitatachibana-mura
Seta-gun Gunma 377(JP)
Inventor: Yamamoto, Tadashi
20-403, 11 Hino 6-chome Konan-ku
Yokohama-shi Kanagawa 233(JP)
Inventor: Toyoshima, Kumao
4-20, Kaminoge 3-chome
Setagaya-ku Tokyo 154(JP)

(74) Representative: von Kreisler, Alek et al
Patentanwälte Von Kreisler-Selting-Werner
Deichmannhaus am Hauptbahnhof
D-5000 Köln 1(DE)

(54) Polypeptide and production thereof.

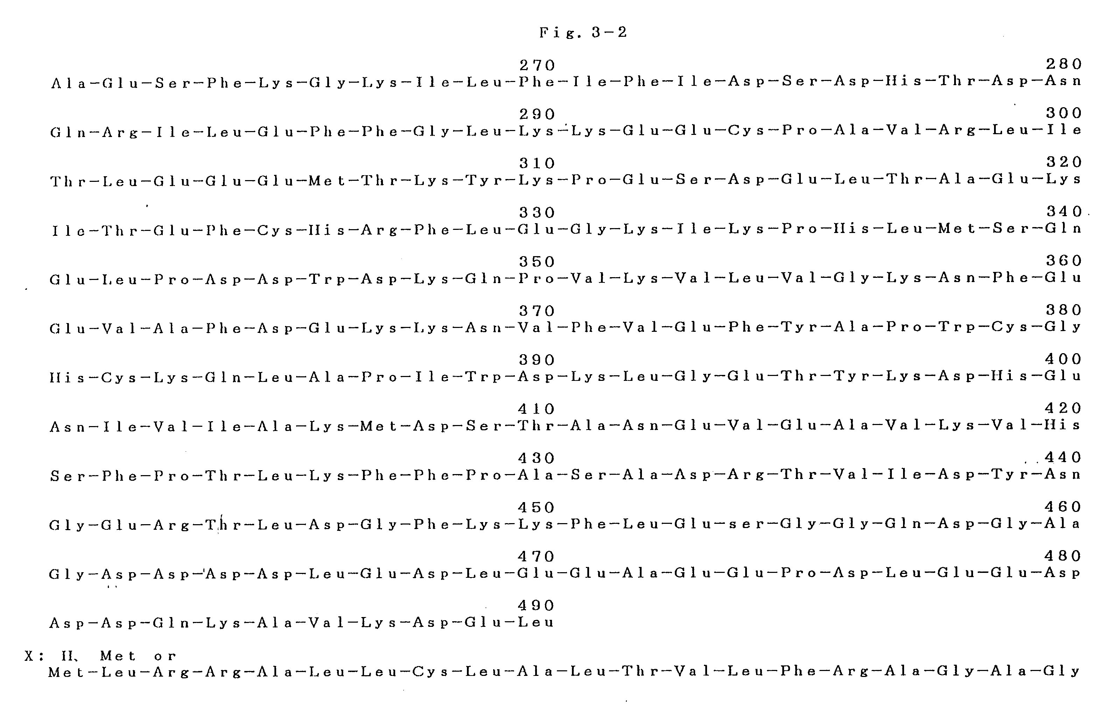

(57) Bovine protein disulfide isomerase (PDI) is disclosed that is a polypeptide containing the amino acid sequence shown in Figure 3. Since the polypeptide has protein disulfide isomerase activity and it catalyzes the exchange reaction between sulfhydryl and disulfide in protein, the polypeptide can be used as a catalyst to re-construct protein having an natural-type disulfide bond from reduced-type protein and oxidized-type protein having unnatural-type disulfide bond.

EP 0 277 563 A1

# POLYPEPTIDE AND PRODUCTION THEREOF

## Field of the invention

The present invention relates to a polypeptide, specifically a polypeptide possessing protein disulfide isomerase activity.

## Description of the Prior Art

It is generally recognized that the formation of a disulfide bond is very important for the retention of the steric structure of protein and for the expression of its activity. In eucaryotic cells, a disulfide bond is formed in the endoplasmic reticulum simultaneously with or immediately after protein translation [J. Biol. Chem., 257, 8847 (1982)]. This disulfide bond is also enzymatically formed in vitro, but the reaction rate is lower than that in vivo, and, in addition, the conditions in vitro do not reflect those in vivo. Based on this fact, Anfinsen et al. hypothesized that a protein such as disulfide interchange protein would function in the formation of a disulfide bond in vivo [J. Biol. Chem. 238, 628 (1963)]. This idea even now remains a hypothesis; however, enzymes which in vitro act on reduced-type or scrambled-type ribonucleases (both of which are of the inactive type) to promote the formation of a native disulfide bond so that the ribonucleases are converted to active-types have been found in various eucaryotes and purified [Biochemistry, 20, 6594(1981); Biochem, J., 213, 225 (1983); Biochem. J., 213, 245 (1983)]. Scrambled-type ribonuclease, which is prepared under denaturing conditions by reduction and re-oxidation, is a mixture of various molecules in which 4 pairs of disulfide bond contained in active-type ribonuclease are recombined randomly [Biochem. J., 213, 235(1983)]. The enzyme which converts inactive-type ribonuclease to an active-type is named protein disulfide isomerase (PDI, EC 5.3.4.1), which is a dimer consisting of 2 identical subunits having a molecular weight of 52000 to 62000, its isoelectric point being 4.0 to 4.5 [Trends in Biochem. Sci., 9, 438 (1984); Methods in Enzymol., 107, 281 (1984)]. The known enzyme protein disulfide reductase (=glutathione insulin transhydrogenase, EC 1.8.4.2) is thought to be a molecule identical with PDI [See Eur. J. Biochem., 32, 27(1973) and Biochemistry, 14, 2115 (1975)].

In 1985, Edman et al. succeeded in cloning the complementary DNA of rat liver PDI and clarified the whole base sequence thereof [Nature, 317, 267(1985)]. Rat liver PDI consists of 489 amino acids and its amino terminus has an added signal peptide consisting of 19 amino acids. Rat liver PDI has also been found to have two regions which are homologous with Escherichia coli thioredoxin, which is known as a cofactor of various oxidation-reduction reactions such as ribonucleotide reductase reaction [Eur. J. Biochem., 6, 475(1968); Pro. Natl. Acad. Sci. USA, 72, 2305 (1975); Methods in Enzymol., 107, 295(1984)], near its amino and carboxyl terminus. Both regions contain a homologous sequence having 2 Cys residues (Trp-Cys-Gly-His-Cys-Lys), which is thought to catalyze PDI reaction via the exchange of disulfide and sulfhydryl. It has also recently been reported that Escherichia coli thioredoxin exhibits PDI activity on reduced-type and scrambled-type ribonuclease as a substrate [Pro. Natl. Acad. Sci. USA, 83, 7643 (1986)].

As to the applicability of PDI, it is thought that PDI may be used for the refolding of recombinant protein which is produced by Escherichia coli. Due to the recent rapid progress of gene engineering technology, mass-production of eucaryotic proteins using Escherichia coli has become possible; however, the thus obtained recombinant proteins may lack native disulfide bonds, or may have mislinked disulfide bonds [EMBO Journal., 4, 775 (1985); Genetic Engineering Vol. 4, edited by Williamson, R., p. 127 (1983)]. A refolding procedure is therefore necessary to obtain active-type recombinant protein having native disulfide bonds. This procedure is now carried out chemically in oxidation-reduction reaction using redox buffer and so on, but it appears that the use of PDI, which may function in vivo, is more suitable for the specific formation of native disulfide bonds, and it is thought of as being particularly efficient, especially for recombinant protein having a great number of disulfide bonds. It is also feasible to introduce the PDI-encoding gene to Escherichia coli having a recombinant DNA, and cause it to act on recombinant protein therein.

In PDI production by conventional methods, it is difficult to obtain the materials, and there is a limitation to the amount of PDI produced; it is therefore desirable that a method enabling the mass-production of PDI with high purity be developed. In rats, the rat liver PDI gene has already been cloned [Nature, 317, 267 (1985)], but there are no reports on the cases in which active-type PDI was obtained as a pure product by expressing the PDI gene in Escherichia coli and so on. In addition, there are no known cases in which the PDI of eucaryotes other than the rat was cloned.

The present inventors have studied the biological functions and chemical properties of the protein which combines with the thyroid hormone T3-

(3,3',5-triiodothyronine, hereinafter briefly referred to as T3), (T3 binding protein, hereinafter briefly referred to as T3BP). It is known that T3BP occurs in cell membranes and cytoplasm of mammalian cells, and T3 receptor, in cell nuclei. The present inventors succeeded in preparing an antibody against T3BP, which was derived from bovine liver cell membrane [Horiuchi, R. and Yamauchi, K., Gunma Symposia on Endocrinology, 23 (Center for Academic Publication Japan, Tokyo; VNU Science Press, BV. Utrecht) pp. 149 to 166 (1986)], and made efforts to use gene engineering techniques to clone the gene which encodes T3BP protein, and found that the cDNA of bovine liver T3BP, which was obtained using the above-mentioned T3BP antibody as a probe, to their surprise, encodes bovine PDI protein.

Summary of the Invention

The purpose of the present invention is to provide: (1) a polypeptide containing the amino acid sequence shown in Figure 3 [hereinafter briefly referred to as bovine PDI] (I), (2) a recombinant DNA containing the base sequence which encodes bovine PDI (II), (3) a transformant resulting from transformation with a vector containing the DNA (II), and (4) a method of producing bovine PDI characterized, that transformant resulting from transformation with a vector containing the DNA (II) is cultured, that bovine PDI is produced and accumulated in the culture and that it is then collected.

Brief Description of the Drawings

Figure 1 illustrates schematic diagrams of the restriction maps of the cDNAs, as cloned respectively in T3BP-1 and T3BP-2, which were obtained in Example 2.

Figure 2 illustrates the base sequence of the cDNA as cloned in T3BP-2 which was obtained in Example 2 and the amino acid sequence deduced from the base sequence.

Figure 3 illustrates the amino acid sequence of bovine PDI.

Figure 4 illustrates the base sequence which encodes bovine PDI.

Figure 5 illustrates a construction scheme of bovine PDI gene expression plasmid for animal cells.

Figure 6 illustrates a construction scheme of bovine PDI gene expression plasmid for Escherichia coli.

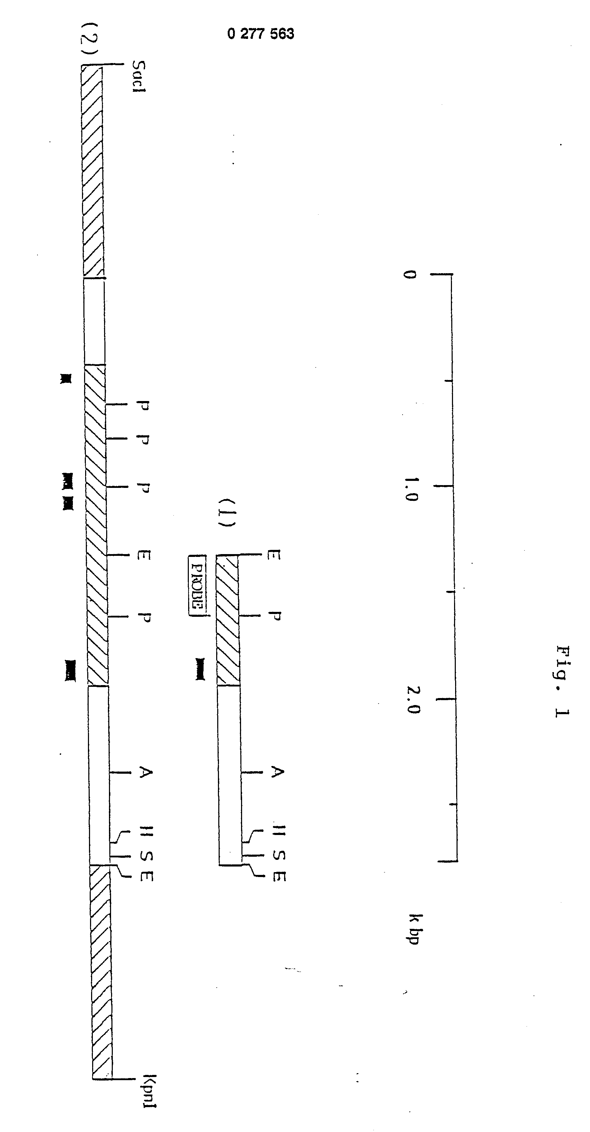

In Figure 1, (1) represents the cDNA which has been cloned in T3BP-1, and (2) represents the cDNA which has been cloned in T3BP-2. The abbreviations A, E, H, P and S respectively represent Aat II, EcoR I, Hinc II, Pvu II and Ssp I; ▨ represents a translation region, and ◳◲ represents a DNA derived from λgt 11. ■ represents a region corresponding to the amino acid sequence which was determined by tryptic peptide mapping of purified T3BP protein.

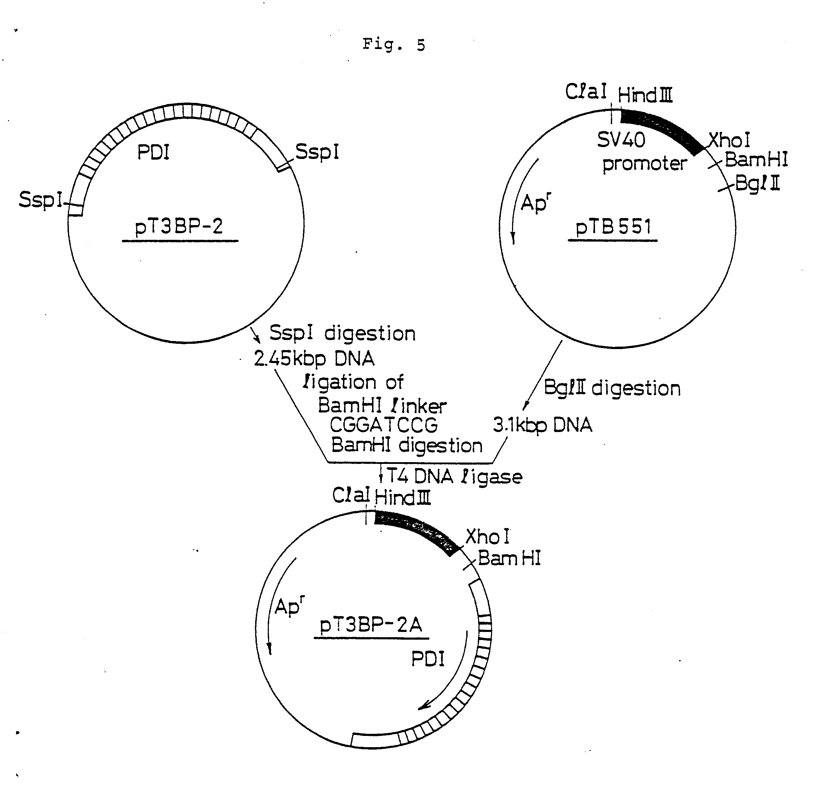

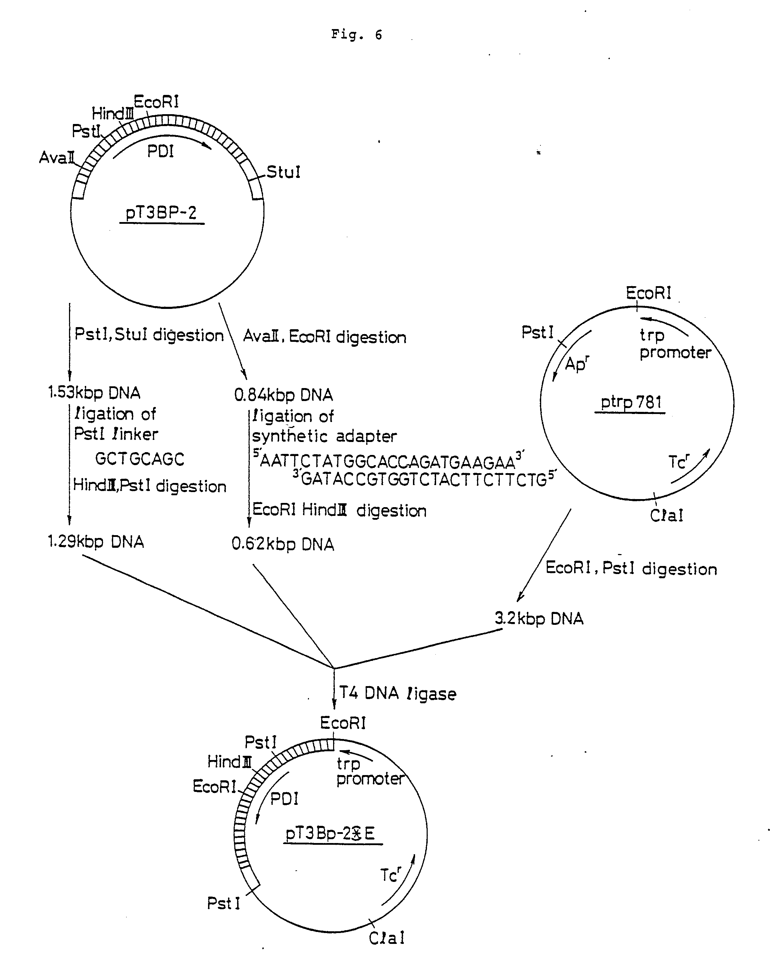

In Figures 5 and 6, ▥ shows a translation region of bovine PDI cDNA, ▭ shows a non-translation region of bovine PDI cDNA and ▬ shows a SV40 promoter region.

Description of the Preferred Embodiments

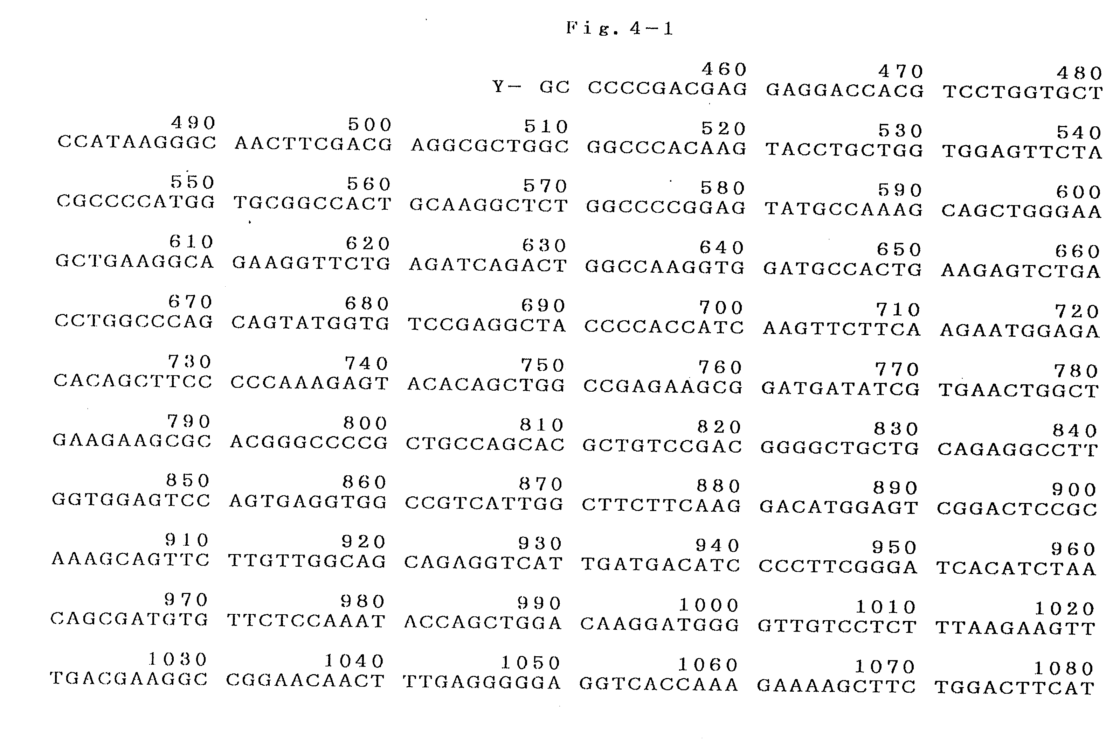

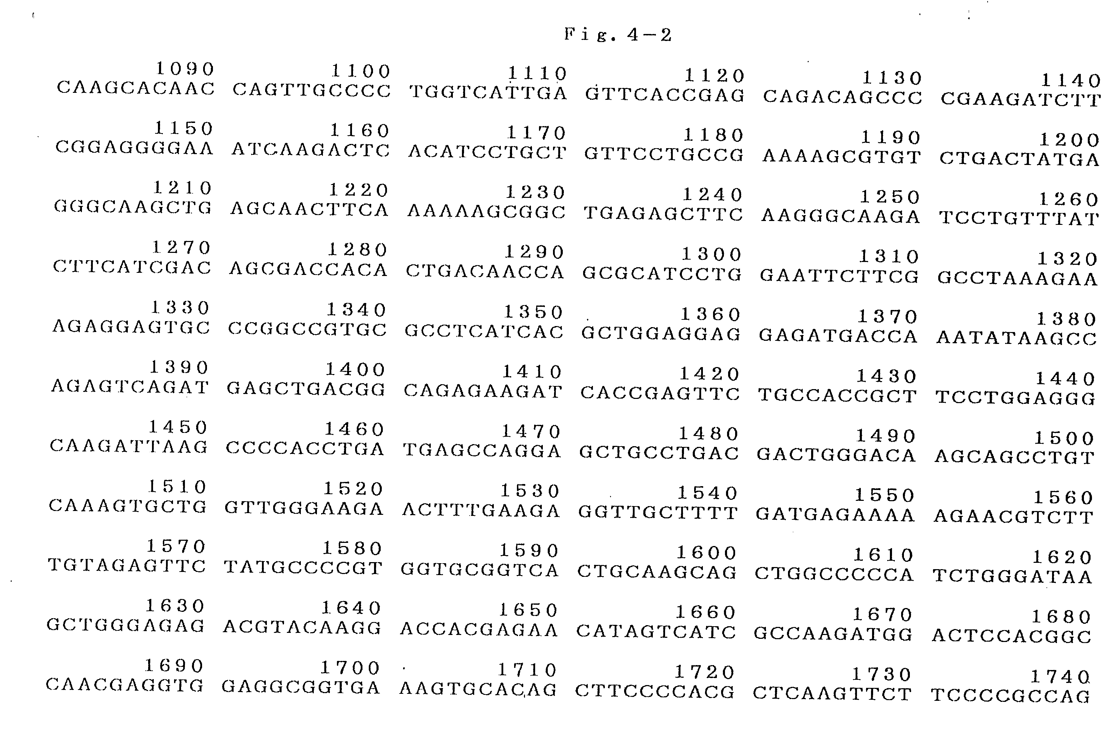

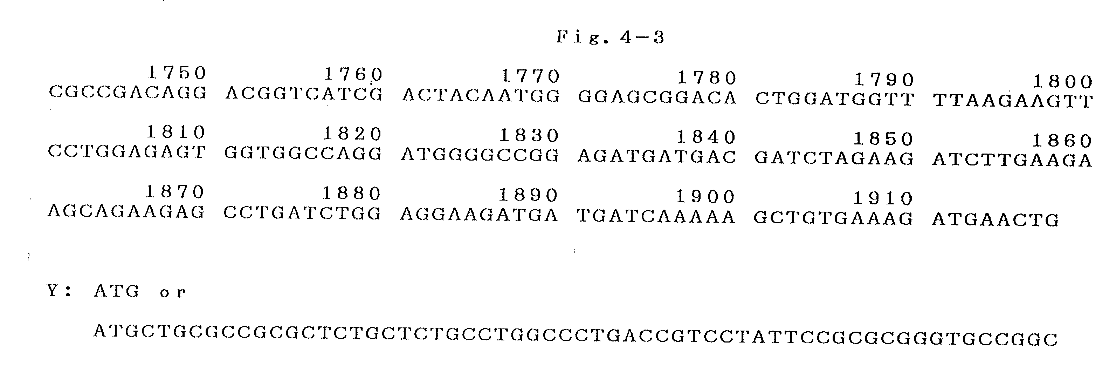

For the above-mentioned DNA (II), any DNA can be used, as long as it contains a base sequence which encodes the amino acid sequence shown in Figure 3; for example, DNA containing the base sequence shown in Figure 4 is preferable.

Bovine PDI as to the DNA, the transformant and the production method of the present invention comprises the PDI (I) represented by the amino acid sequence shown in Figure 3 as a portion thereof.

The expression vector containing the DNA having the base sequence which encodes bovine PDI polypeptide in the present invention can be, for example, produced by (i) separating an RNA which encodes bovine PDI; (ii) synthesizing from the said RNA a single-stranded complementary DNA (cDNA), and then synthesizing a double-stranded DNA; (iii) inserting the double-stranded complementary DNA thus obtained to a phage or plasmid; (iv) transforming a host with the recombinant phage or plasmid thus obtained; (v) isolating from the transformant thus obtained the phage or plasmid containing the desired DNA by an appropriate method, for example, immunoassay using anti-T3BP antibodies; (vi) cutting out the desired cloned DNA from the isolated phage or plasmid , and (vii) ligating the said cloned DNA downstream from the promoter in a vehicle.

The RNA which encodes bovine PDI can be obtained from various bovine PDI-producing cells, for example, bovine hepatocytes.

Methods of preparing RNA from bovine PDI-producing cells include the guanidine thiocyanate method [Chirgwin, J.M. et al.; Biochemistry, 18, 5294 (1979)].

Using the thus obtained RNA as a template, a cDNA is synthesized with use of a reverse transcriptase in accordance with, for example, the methods of Okayama, H. et al. [Mol. Cell. Biol., 2, 161 (1982); Mol. Cell. Biol., 3, 280 (1983)] or the method of Hoffman, B.J. [Gene, 25, 263 (1983)], and the obtained cDNA is inserted in a plasmid or phage.

For examples of plasmids in which cDNA is inserted, see, pBR322 [Gene, 2, 95 (1977)], pBR325 [Gene, 4, 121 (1978)], pUC12 [Gene, 19, 259 (1982)] and pUC 13 [Gene, 19, 259 (1982)], which are derived from Escherichia coli, and pUB110 [Biochem. Biophys. Res. Commun., 112, 678 (1983)], which is derived from Bacillus subtilis. However, any other plasmid can be used, as long as it is replicable and held in a host. For examples of phage vectors in which cDNA is inserted, see, λgt 11 [Young, R., and Davis, R.; Proc. Natl. Acad. Sci. USA, 80, 1194 (1983)]etc., but any other can be used, as long as it is capable of proliferating in the host.

For examples of methods of inserting cDNA in a plasmid vector, see the methods described in "Molecular Cloning", Maniatis, T., et al., Cold Spring Harbor Laboratory, p. 239 (1982). For examples of methods of inserting cDNA in a phage vector, see the method of Hyunh, T. V., et al. [DNA Cloning, A Practical Approach, 1, 49 (1985)]. The plasmid or phage vector thus obtained is introduced in an appropriate host, for example, Escherichia coli.

As examples of useful Escherichia coli strains, there may be mentioned Escherichia coli K12 DHl [Proc. Natl. Acad. Sci. USA, 60, 160(1968)], JM103 [Nucl. Acids, Res., 9, 309 (1981)], JA221 [J. Mol. Biol., 120, 517 (1978)], HB101 [J. Mol. Biol., 41, 459 (1969)] and C600 [Genetics, 39, 440 (1954)].

As examples of useful Bacillus subtilis strains, there may be mentioned Bacillus subtilis MI114 [Gene, 24, 255 (1983)] and 207-21 [J. Biochem., 95, 87 (1984)].

For examples of methods of transforming a host with a plasmid, see the calcium chloride method or calcium chloride/rubidium chloride method described in "Molecular Cloning", Maniatis, T. et al; Cold Spring Harbor Laboratory, p. 249 (1982), etc. Phage vector can be, for example, introduced to grown Escherichia coli by the in vitro packaging method.

The desired clone is selected from the transformants thus obtained by a well-known method such as colony hybridization [Gene, 10, 63 (1980)], plaque hybridization [Science, 196, 180 (1977)] or the DNA base sequence determination method [Proc. Natl. Acad. Sci. USA, 74, 560 (1977)]; Nucl. Acids, Res., 9, 309 (1981)].

A microorganism carrying a vector having the cloned DNA containing the base sequence which encodes bovine PDI is thus obtained.

The plasmid pT3BP-2, which is carried by Escherichia coli K12 DH5α/pT3BP-2 which was obtained in Example 2 below, has a DNA containing the base sequence which encodes bovine PDI (I). A portion of the said DNA is thought to encode bovine PDI(I). Figure 1 shows the restriction sites of the said DNA. As shown in Figure 1, the said DNA has a total length of about 2.8 Kbp, and it is cleaved into fragments with restriction enzymes Aat II, EcoRI, HincII, PvuII and SspI.

The plasmid or phage vector is then isolated from the said microorganism.

As methods of such isolation, there may be mentioned the alkali method [Birmboim, H.C., et al.; Nucl. Acids Res., 7, 1513 (1979)] and so on.

The above-mentioned plasmid or phage vector having a cloned DNA containing the base sequence which encodes bovine PDI can be used directly or, if desired, after digestion with restriction enzyme, depending on the objective.

The cloned gene can be ligated downstream from the promoter in a suitable vehicle(vector) for the expression of the gene to obtain an expression vector.

As vectors, there may be mentioned the above-mentioned plasmids derived from Escherichia coli(e.g., pBR322, pBR325, pUC12 and pUC13), plasmids derived from Bacillus subtilis - (e.g., pUB110, pTB5 and pC194), plasmids derived from yeasts (e.g., pSH19 and pSH15), bacteriophages such as λphage, and animal viruses such as retrovirus and vaccinia virus.

The said gene may have ATG as a translation initiation codon at its 5'-terminus and have TAA, TGA, or TAG as a translation termination codon at its 3'-terminus. For the expression of the said gene, a promoter is ligated to the upstream thereof. Any promoter can be used for the present invention, as long as it is appropriate for the host to be used for the gene expression.

When the host for transformation is Escherichia coli, trp promoter, lac promoter, rec promoter, $\lambda P_L$ promoter, lpp promoter and so on are preferable; when the host is Bacillus subtilis, SP 01 promoter, SP 02 promoter, penP promoter, etc. are preferable; and when the host is yeast, PHO 5 promoter, PGK promoter, GAP promoter, ADH promoter, etc. are preferable. It is especially preferable that the host be Escherichia coli, and the promoter be trp or λ $P_L$ promoter.

When the host is an animal cell, there may be mentioned promoters derived from SV40, promoters from retrovirus and so on; the use of a promoter derived from SV40 is particularly preferable.

Using the vector containing the DNA(II) thus obtained, transformants are prepared.

As hosts, there may be mentioned procaryotes such as Escherichia coli, Bacillus subtilis and actionomycetes; and eucaryotes such as yeasts, fungi and animal cells.

As specific examples of the above-mentioned Escherichia coli and Bacillus subtilis, there may be mentioned the same strains as above.

As specific examples of the above-mentioned yeasts, there may be mentioned Saccharomyces cerevisiae AH22, AH22R , NA87-11A, DKD-5D and so on.

As specific examples of the above-mentioned animal cells, there may be mentioned the monkey cell COS-7, Vero, the Chinese hamster ovary cell (CHO) and mouse L-cells.

The transformation of the above-mentioned Escherichia coli is carried out in accordance with, for example, the method described in Proc. Natl. Acad. Sci. USA, 69, 2110 (1972), Gene, 17, 107 (1982), and so on.

The transformation of Bacillus subtilis is carried out in accordance with, for example, the method described in Molec. Gen. Genet., 168, 111 (1979) etc.

The transformation of yeast is carried out in accordance with, for example, the method described in Proc. Natl. Acad. Sci. USA, 75, 1929 (1978).

The transformation of animal cells is carried out in accordance with, for example, the method described in Virology, 52, 456 (1973).

A transformant resulting from transformation with a vector containing the DNA (II) is thus obtained.

In culturing transformants whose host is Escherichia coli, Bacillus subtilis, an actynomycete, a yeast, or a fungus, it is appropriate to use a liquid medium for the cultivation, which is supplemented with carbon sources, nitrogen sources, minerals, and other substances which are essential to the growth of the transformants. Carbon sources include glucose, dextrin, soluble starch and sucrose; nitrogen sources include organic or inorganic substances such as ammonium salts, nitrates, corn steep liquor, peptone, casein, meat extract, soybean cake and potato extract; minerals include calcium chloride, sodium dihydrogenphosphate and magnesium chloride.

It is desirable that the pH of medium be about 5 to 8.

When the host is Escherichia coli, the use of, for example, the M9 medium [Miller, J.; J. Exp. Mol. Genet., p.431, Cold Spring Harbor Laboratory, New York, (1972)], which contains glucose and Casamino Acids, is preferable. The cultivation is normally carried out at about 14 to 43°C for about 3 to 24 hours, and, where necessary, aeration and/or agitation are added.

When the host is Bacillus subtilis, cultivation is normally carried out at about 30 to 40°C for about 6 to 24 hours, and, where necessary, aeration and/or agitation are added.

In culturing transformants whose host is a yeast, Bulkholder's minimum medium [Bostian, K.K. et al.; Proc. Natl. Acad. Sci. USA, 77, 4505 (1980)] can be used for the cultivation. It is preferable that the medium be adjusted to pH 5 to 8. The cultivation is normally carried out at about 20 to 35°C for about 24 to 72 hours, and, if necessary, aeration and/or agitation are added.

In culturing transformants whose host is an animal cell, media which can be used include the MEM medium [Science, 122, 501 (1952)] supplemented with 5 to 20% fetal bovine serum, the DMEM medium [Virology, 8, 396 (1959)], the RPMI1640 medium [J. Am. Med. Assoc., 199, 519 (1967)], the 199 medium [Proc. Soc. Exp. Biol. Med., 73, 1 (1950)]. It is preferable that pH be about 6 to 8. The cultivation is normally carried out at about 30 to 40°C for about 15 to 60 hours, and, if necessary, aeration and/or agitation are added.

PDI protein of the present invention is produced and accumulated in or outside cells. In extracting intracellular PDI from the culture, there may be used appropriate known methods can be used. For example, a supernatant containing PDI is obtained by collecting cells by a known method after cultivation, suspending the cells in a buffer solution containing a protein denaturant such as guanidine hydrochloride or urea or in a buffer solution containing a surfactant such as Triton X-100, then centrifuging the suspension; or a supernatant containing PDI is obtained by disrupting the cells by ultrasonication, treatment with enzyme such as lysozyme, or freezing-thawing, then centrifuging the cell fraction.

Separation and purification of PDI from such supernatant or PDI produced and accumulated outside cells can be achieved by carrying out well known methods of separation and purification in appropriate combination. As examples of such known methods of separation and purification, there may be mentioned methods based on the difference in solubility, such as salting-out and solvent precipitation; methods based mainly on the difference in molecular weight, such as dialysis, ultrafiltration, gel filtration and SDS-polyacrylamide gel electrophoresis; methods based on the difference in electric charge, such as ion-exchange chromatography; methods based on specific affinity, such as affinity chromatography; methods based on the difference in hydrophobicity, such as reversed-phase high performance liquid chromatography; and methods based on the difference in isoelectric point, such as isoelectric electrophoresis. Specifically, ion-exchange chromatography using diethylaminoethyl (DEAE) cellulose, carboxymethyl (CM) cellulose or the like is efficient for the purification of bovine PDI protein, because this protein is an acidic protein.

Activity of PDI protein thus obtained (PDI activity) can be determined by measuring the rate of conversion of reduced-type and scrambled-type ribonucleases as a substrate to active-type ribonucleases [Biochem. J., 159, 377 (1976); Proc. Natl. Acad. Sci. USA, 83, 7643 (1986)].

The bovine PDI protein of the present invention catalyzes the exchange reaction between sulfhydryl and disulfide in any desired protein to form a natural-type disulfide bond with the maximum stability. Specifically, the said protein acts on reduced protein in the presence of dissolved oxygen or a mixture of oxidized-type glutathione (GSSG) and reduced-type glutathione (GSH) to catalyze the reaction by which a natural-type disulfide bond is formed, or acts on oxidized-type protein having a disulfide bond, specifically a unnatural-type disulfide bond to catalyze the reaction by which a natural-type disulfide bond is re-formed.

In producing protein having a disulfide bond in its molecules by genetic engineering techniques, it is therefore possible to use bovine PDI protein to form at high efficiency a natural-type disulfide bond in the protein molecules, specifically when the host of the recombinant DNA is a procaryote such as Escherichia coli or Bacillus subtilis. As examples of such proteins, there may be mentioned cytokines such as interferon-α, interferon-β interferon-γ, interleukin-1, interleukin-2, B-cell growth factor (BGF), B-cell differentiation factor (BDF), macrophage activating factor (MAF), lymphotoxin (LT) and tumor necrosis factor (TNF); peptide protein hormones such as transforming growth factor (TGF), erythropoiethin, epidermal growth factor (EGF), fibroblast growth factor (FGF), insulin and human growth hormone; pathogenic microbial antigen proteins such as hepatitis B virus antigen; enzymes such as peptidase and lysozyme; and blood protein components such as human serum albumin and immunoglobulin.

PDI protein used herein may have been purified, or may have been partially purified; the purpose is accomplished only by reacting the said PDI protein directly with the above-mentioned desired protein produced and accumulated in or outside cells, or with a purified standard sample thereof. The reaction may also be carried out using a transformant prepared by the double infection of the host containing a recombinant DNA which encodes the said PDI protein with a recombinant DNA which encodes the above mentioned desired protein.

Bovine PDI of the present invention catalyzes the exchange reaction between sulfhydryl and disulfide in protein, and can be used to re-construct protein having natural-type disulfide bonds from reduced-type protein and oxidized-type protein having unnatural-type disulfide bonds.

The definitions of the abbreviations used in the present specification, the claims and the abstract are as shown in Table 1.

Table 1

DNA Deoxyribonucleic acid
cDNA : Complementary deoxyribonucleic acid
A : Adenine
T : Thymine
G : Guanine
C : Cytosine
RNA : Ribonucleic acid
mRNA : Messenger RNA
dATP : Deoxyadenosine triphosphate
dTTP : Deoxythmidine triphosphate
dGTP : Deoxyguanosine triphosphate
dCTP : Deoxycytidine triphosphate
ATP : Adenosine triphosphate
EDTA : Ethylenediaminetetraacetic acid
SDS : Sodium dodecylsulfate
Gly : Glycine
Ala : Alanine
Val : Valine
Leu : Leucine
Ile : Isoleucine
Ser : Serine
Thr : Threonine
Cys : Cysteine
Met : Methionine
Glu : Glutamic acid
Asp : Aspartic acid
Lys : Lysine
Arg : Arginine
His : Histidine
Phe : Phenylalanine
Tyr : Tyrosine
Trp : Tryptophan
Pro : Proline
Asn : Asparagine
Gln : Glutamine

In PDI protein of the present invention, any part (up to about 5% ratio) of the amino acid sequence may be modified (addition, elimination, substitution by other amino acids, etc.).

The present invention will hereinafter be described in detail with the following examples. It is understood of course that these examples are not intended to limit the scope of the invention.

Example 1

Preparation of cDNA Library Deriving from Bovine Liver mRNA

RNA was extracted from bovine liver by the guanidine isothiocyanate method [Chirgwin, J. M. et al.; Biochemistry, 18, 5294 (1979)]. This RNA was subjected to oligo dT cellulose column chromatography [Aviv and Leder, Proc. Natl. Acad.

Sci, USA, 69, 1408 (1972)] to purify poly(A) RNA.

Using this poly(A) RNA as a template, cDNA was synthesized by the method of Gubler, U. and Hoffman, B. J. [Gene, 25, 263 (1983)]. An EcoRI linker was then linked to the above-mentioned cDNA in accordance with the method of Huynh, T. V. et al. [DNA Cloning I, A Practical Approach, 1, 49 (1985)], and this was cloned at the EcoRI site of λgtII to prepare a cDNA library.

Example 2

Isolation of Plasmid Containing Bovine PDI cDNA and Determination of the Base Sequence Thereof

Escherichia coli Y1096 was infected with the λgtII cDNA library obtained in Example 1, and then inoculated over an L-Broth soft agar plate. When plaques appeared, a nitrocellulose membrane containing IPTG (isopropylthiogalactoside) was placed on the plate, and incubated for 3 hours. The nitrocellulose membrane was separated, rinsed with a TBS-buffered solution (50mM Tris, pH 7.9, 150mM NaCl), and then immersed in a 3% gelatin solution.

The so treated nitrocellulose membrane was immersed in a solution of antibodies against T3-conjugated protein (anti-BLT3R) [Horiuchi, R. and Yamauchi, K.; Gunma Symposia on Endocrinology, 23, p.149 (1986)] to conduct antigen-antibody reaction. This membrane was then rinsed with distilled water and a TBS-buffered solution, and then reacted with secondary antibodies, to select the coloring positive clone λgtII T3BP-1. Using EcoRI, cDNA was cut out from λgtII T3BP-1, and it was recloned at the EcoRI site of the plasmid pUC19 [Gene, 33, 103 (1985)] to prepare the plasmid pT3BP-1. Of the cDNA region contained in the plasmid thus obtained, the EcoRI-PvuII restriction fragment was used as a DNA probe for re-screening of $5 \times 10^5$ clones of the cDNA library described in Example 1 to select positive clones.

Using SacI-KpnI, cDNA was cut out from one of the clones thus obtained, λgtII T3BP-2, and it was re-cloned to the SacI-KpnI site of the plasmid pUC19 to prepare plasmid pT3BP-2. The EcoRI recognition site at the 5'-terminus of the cDNA which was cloned to λgtII T3BP-2 was destroyed.

E. coli DH5α was transformed with plasmid pT3BP-2, and the resulting transformant E. coli DH5α/pT3BP-2 was subjected to extraction by the alkali method [Birnboim, H. C. and Doly, J.; Nucl. Acids Res., 7, 1513 (1979)] to purify plasmid pT3BP-2. This plasmid was found to contain a cDNA region having about 2.8 Kbp in total length. Figure 1 shows a schematic diagram of the restriction map thereof.

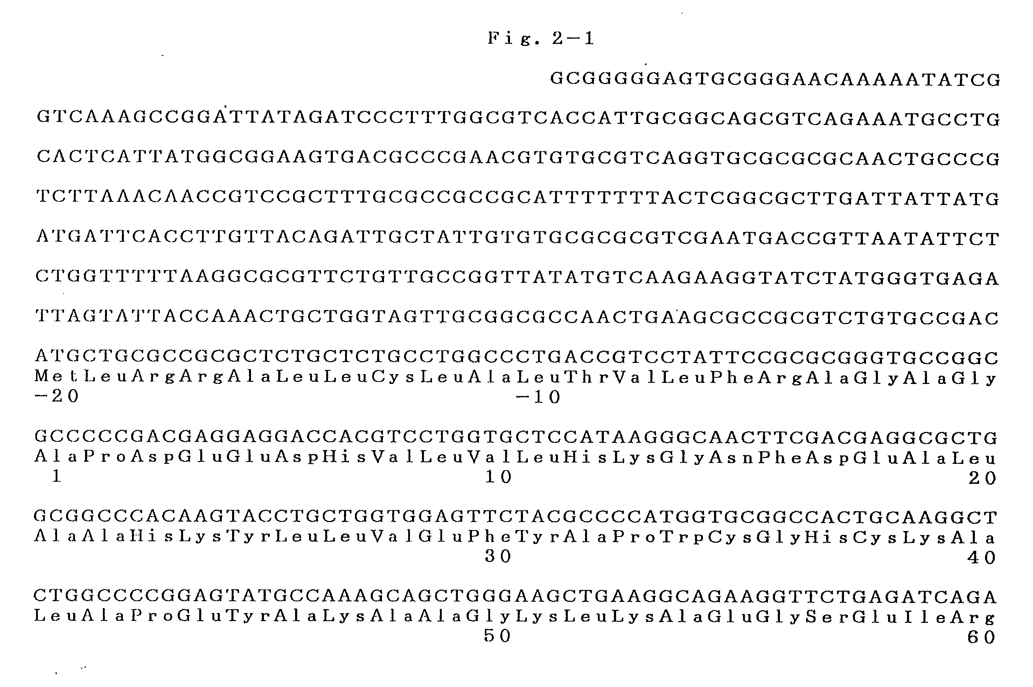

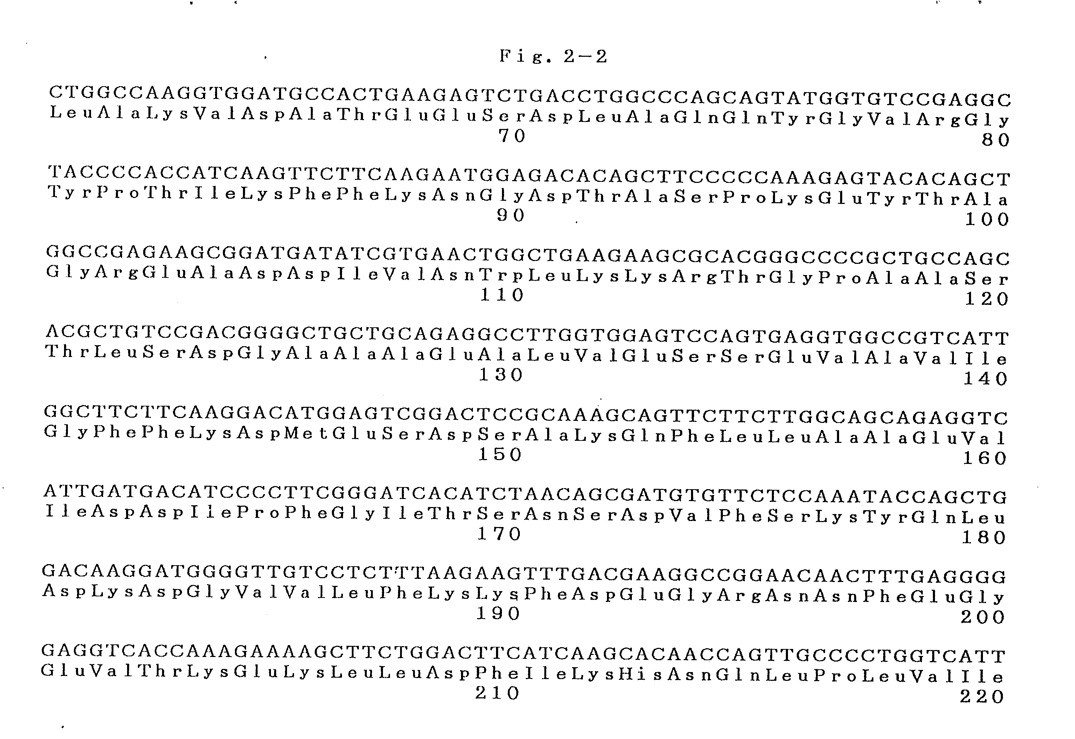

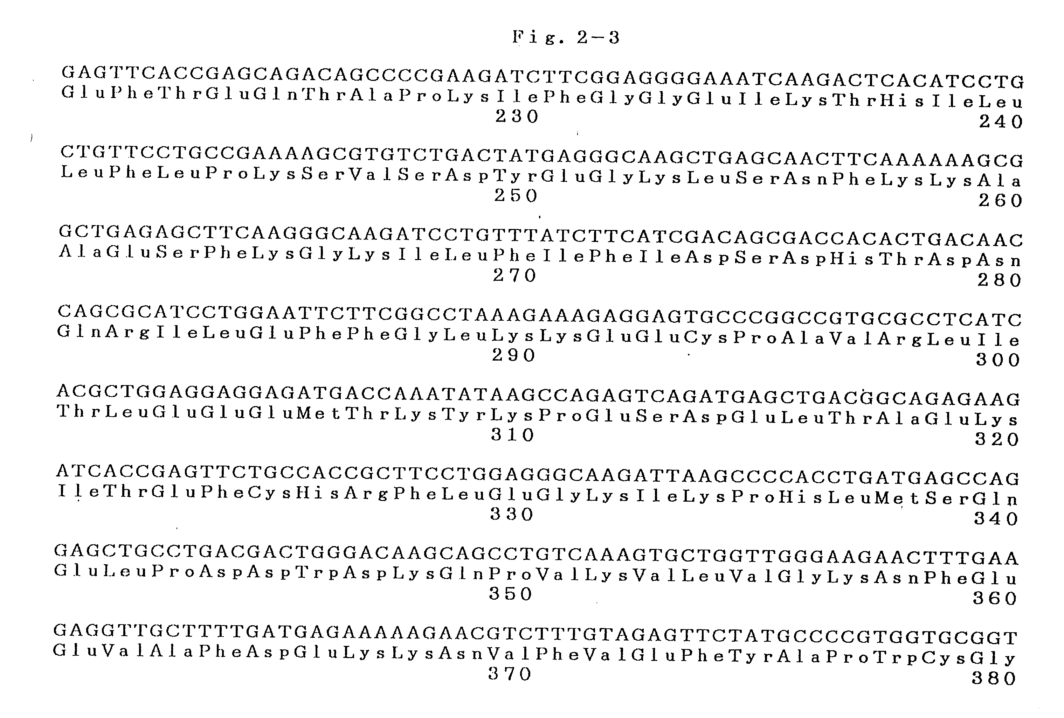

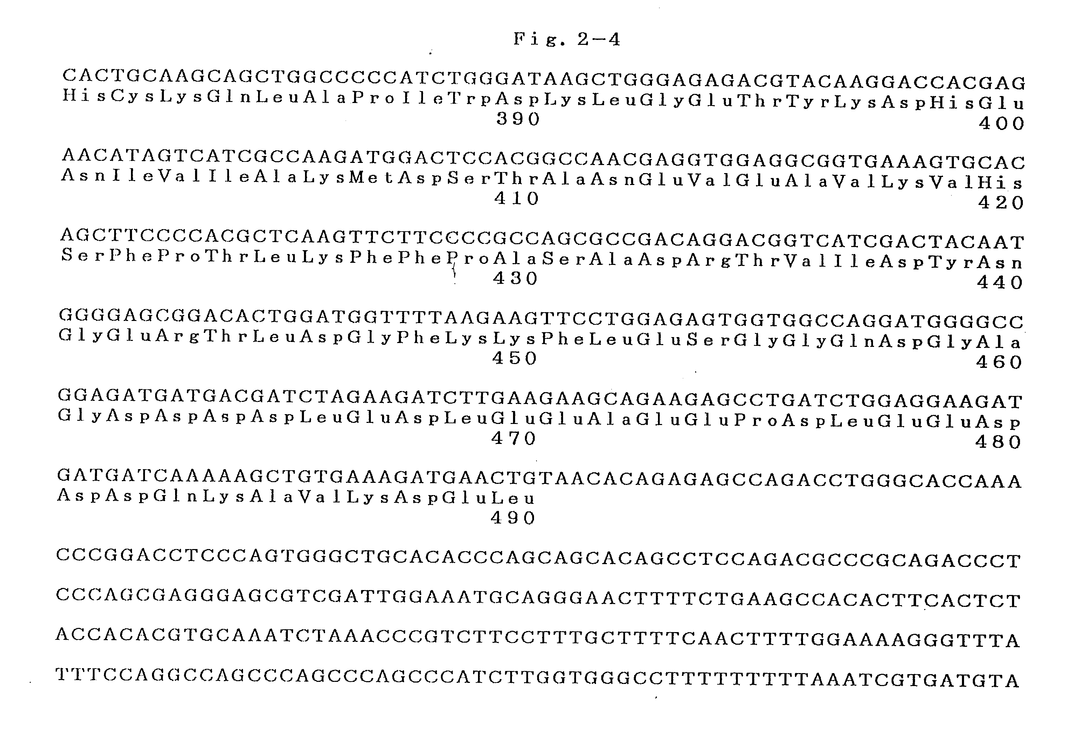

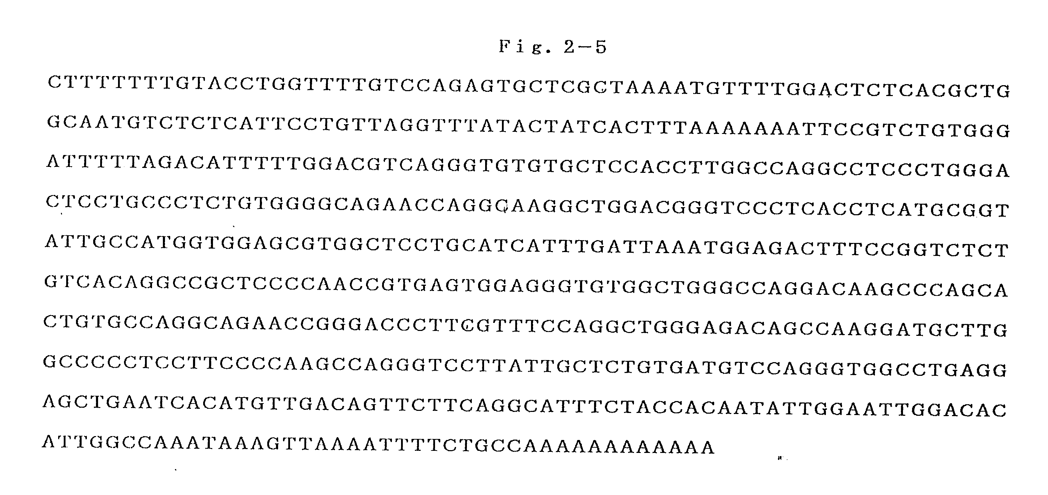

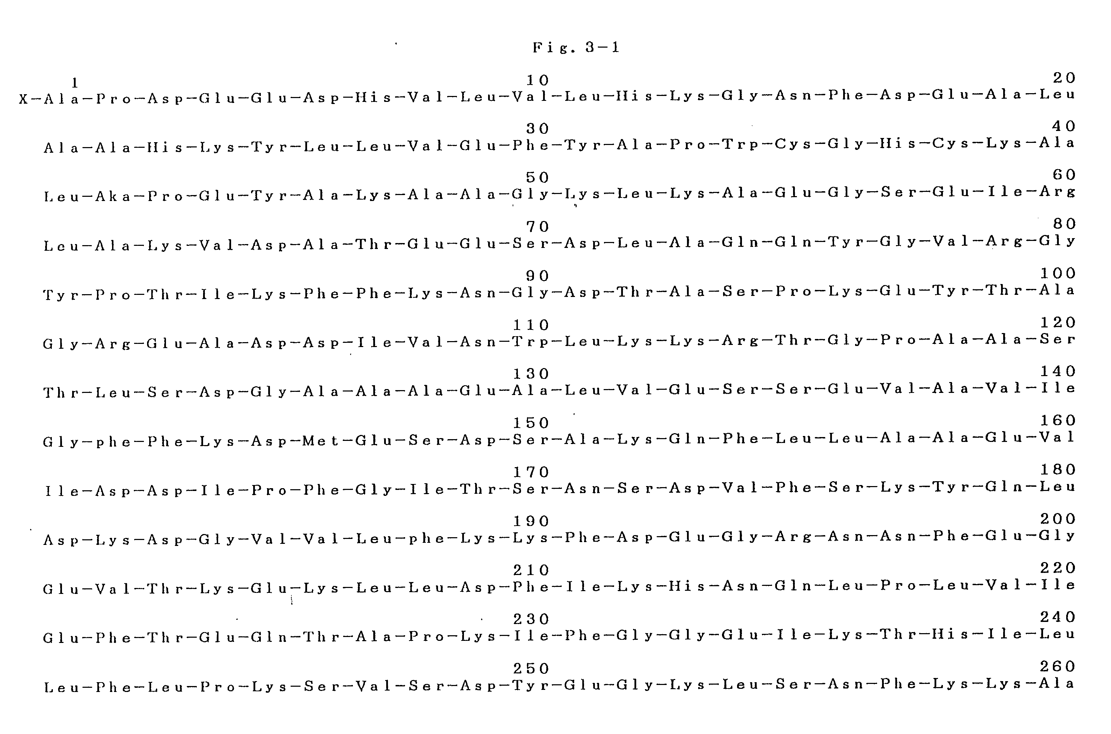

The base sequence of this cDNA region was determined by the dideoxynucleotide chain termination method [Messing, J., et al.; Nucl. Acids Res., 9, 309(1981)]. Figure 2 shows the amino acid sequence deduced from the determined base sequence.

The amino acid sequence of the said polypeptide had a homology with that of rat PDI at a level of more than 90%, but it showed no homology with the amino acid sequence of thyroxine binding globulin (TBG), which is capable of binding with thyroid hormone, thyroxin binding prealbumin (TBPA) or C-erbA protein.

Escherichia coli K12 DH5α carrying plasmid pT3BP-2 (Escherichia coli K12 DH5α/pT3BP-2) has been deposited under the accession number IFO 14563 at the Institute for Fermentation, Osaka (IFO) and under the accession number FERM BP-1595 at the Fermentation Research Institute, Agency of Industrial Science and Technology, Japan.

Example 3

Construction of Expression Plasmid for Animal Cell

Plasmid pT3BP-2 DNA is digested with restriction enzyme SspI to separate 2.45 Kbp cDNA. This DNA is ligated with a BamHI linker (CGGATCCG) by T4 DNA ligase and the obtained DNA is digested with BamHI. Then, the obtained DNA is mixed with a DNA obtained by cleaving plasmid pTB551 with BglII, and the mixture is treated with T4 DNA ligase to construct plasmid pT3BP-2A (Fig. 5). The above-described plasmid pTB551 may be obtained by changing the EcoRI recognition site of plasmid pTB389 to the BglII recognition site. The plasmid pTB389 is obtained by changing the PstI cleaving site, which is located at the 5'-terminus of the Interlenkin-2 gene region of plasmid pTB106 disclosed in Japanese Unexamined Patent Publication No. 61-63282 (63282/1986), to an EcoRI site, and the BamHI cleaving site at the 3'-terminus of the IL-2 gene region to an EcoRI site, and by removing the Interleukin-2 gene region.

Example 4

Expression of Gene coding for Bovine PDI in Animal Cell

Monkey COS-7 cells are cultured in monolayer in DMEM medium containing 10% fetal calf serum (FCS), followed by exchanging the medium for the same medium. After 4 hours from the exchange, calcium phosphate gel containing 10 μg of the DNA of plasmid pT3BP-2A, which is prepared according to the known method [Graham, et al., Virology, 52, 456(1973)], is added to the cells, whereby COS-7 cells infected with pT3BP-2A are obtained. After 4 hours, the COS-7 cells infected with pT3BP-2A described above are treated with glycerol, and cultivation is continued in the medium containing 0.5% FCS.

After 48 hours, the COS-7 cells infected with pT3BP-2A are fixed with PBS containing 3.5% formalin at room temperature for 15 minutes. Then, the COS-7 cells are treated with PBS containing 0.1% saponine at room temperature for 10 minutes, followed by the reaction with the above-described anti bovine T3BP rabbit antibodies at 4°C for 2 hours. After washing off the unreacted antibodies, the cells are reacted with FITC labeled-anti rabbit IgG sheep antibodies overnight and observed with fluorescence microscope.

As a result, in the case of the cells induced with the bovine PDI gene, strong fluorescene is observed as compared to the control COS cells and it is proven that the bovine PDI protein is synthesized in the infected COS-cells.

## Example 5

Construction of Bovine PDI Gene Expression Plasmid for Escherichia coli

Plasmid pT3BP-2 containing bovine PDI cDNA, which is obtained in Example 2, is cleaved with restriction enzymes AvaII and EcoRI to obtain a 0.84 Kbp DNA fragment containing the first half of the region coding for bovine PDI. To the DNA fragment, the following synthesized oligonucleotides that have been already subjected to phosphorylation reaction are ligated by T4DNA ligase:

$$5'AATTCTATGGCACCAGATGAAGAA^{3'} \text{ and}$$

$$5'GTCTTCTTCATCTGGTGCCATAG^{3'}$$

followed by cleaving with EcoRI and Hind III to separate and purify a 0.62 Kbp DNA fragment.

Plasmid pT3BP-2 is cleaved with StuI and PstI to obtain a 1.53Kbp DNA fragment containing the latter half of the region coding for PDI. To the DNA is ligated a PstI linker (GCTGCAGC) by T4 DNA ligase and the producut is cleaved with PstI and HindIII to prepare a 1.29 Kbp DNA fragment.

DNA of plasmid ptrp 781 containing a trp promoter [Kurokawa, T et al., Nucleic Acids Res., 11: 3077-3085(1983)] is cleaved with EcoRI and pstI to separate an approximately 3.2 Kbp DNA containing a trp promoter, a tetracycline resistant gene and a plasmid replication origin.

The above-described 0.62 Kbp EcoRI-HindIII fragment containing the gene coding for bovine PDI, the 1.29 Kbp HindIII-PstI DNA fragment and the 3.2 Kbp EcoRI-PstI DNA fragment are ligated together by T4 DNA ligase to construct bovine PDI expression plasmid pT3BP-2E (Fig.6). Escherichia coli K12 MM294 is transformed with the above-obtained plasmid to obtain a strain Esherichia coli K12 MM294/pT3BP-2E.

## Example 6

Label of Escherichia coli with $^{35}$S-methionine and Immuno Precipitation Reaction

Escherichia coli MM294 containing expression plasmid pT3BP-2E is cultured in M9 medium containing 8 μg/ml of tetracycline, 0.2% Casamino acid and 1% glucose at 37°C. When Klett value reaches at 200, IAA ($3\beta$-indolylacrylic acid) is added to the medium up to a concentration of 25 μg/ml. After 2 hours from the addition, $^{35}$S-methionine is added to a concentration of 15 μCi/ml to label a sythesized protein for 30 minutes. After labeling, the cells are collected and washed with 0.15 M NaCl solution. The cells are suspended in a 10 mM Tris-HCl (pH 8.0) solution containing 10% sucrose, of which volume is equivalent to one-fifth of the culture solution. Phenylmethylsulfonyl fluoride (PMSF), NaCl and EDTA are add to the suspension in concentrations of 1 mM, 0.2 M and 10 mM respectively, and then lysozyme is added up to a concentration of 150 μg/ml. The obtained mixture is reacted at 0°C for 1 hour. The suspension is treated at 31°C for 2 minutes, followed by short ultrasonication (for approximate 20 seconds) and then centrifugation to obtain a cell extract. To the extract are added anti bovine T3BP rabbit antibodies [Horiuchi, R. and Yamauchi, K., Gunma Symposia on Endocrinology, 23, 149(1986)] and the mixture is reacted at 4°C overnight. Staphylococcal cells (protein A) are added to the mixture and antigen-antibody conjugated materials are bound to the cells at 0°C for 3 hours. The cells are subjected to a few times of centrifugation and washing with a solution [50 mM Tris-HCl pH 7.4, 5 mM EDTA, 150 mM NaCl, 0.05% Nonidet P-40 (Shell Oil), 1 mg/ml ovalbumin], rollowed by treating at 100°C for 5 minutes in an electrophoresis solution (0.0625M Tris-HCl, pH 6.8, 2% SDS, 5% 2-mercaptoethanol, 0.001% Bromophenol Blue, 10% glycerol) to elute the conjugated materials. The obtained immunoprecipitate is analyzed by 10 to 20% gradient SDS poly-acrylamide gel electrophoresis. After the electrophoresis, the gel is fixed with 50% trichloroacetic acid and radioautogram thereof is taken by fluorography. As a result, it is proven that bovine PDI having an expected size of approximately 60 KDa is produced in E. coli strain MM294/pT3BP-2E containing the expression plasmid.

## Claims

1. A PDI polypeptide containing the amino acid sequence shown in Figure 3.

2. A recombinant PDI DNA containing the base sequence which encodes the PDI amino acid sequence shown in Figure 3.

3. The recombinant PDI DNA as claimed in claim 2 wherein the base sequence is shown in Figure 4.

4. A transformant resulting from transformation with a vector containing the nucleotide sequence which encodes the PDI amino acid sequence shown in Figure 3.

5. A method of producing the PDI polypeptide containing the PDI amino acid sequence shown in Figure 3, characterized in that a transformant resulting from transformation with a vector containing the nucleotide sequence which encodes the PDI amino acid sequence shown in Figure 3 is cultured to produce and accumulate said polypeptide in the culture, which is then collected.

Fig. 1

Fig. 2-1

GCGGGGGAGTGCGGGAACAAAAATATCG

GTCAAAGCCGGATTATAGATCCCTTTGGCGTCACCATTGCGGCAGCGTCAGAAATGCCTG

CACTCATTATGGCGGAAGTGACGCCCGAACGTGTGCGTCAGGTGCGCGCGCAACTGCCCG

TCTTAAACAACCGTCCGCTTTGCGCCGCCGCATTTTTTTACTCGGCGCTTGATTATTATG

ATGATTCACCTTGTTACAGATTGCTATTGTGTGCGCGCGTCGAATGACCGTTAATATTCT

CTGGTTTTTAAGGCGCGTTCTGTTGCCGGTTATATGTCAAGAAGGTATCTATGGGTGAGA

TTAGTATTACCAAACTGCTGGTAGTTGCGGCGCCAACTGAAGCGCCGCGTCTGTGCCGAC

ATGCTGCGCCGCGCTCTGCTCTGCCTGGCCCTGACCGTCCTATTCCGCGCGGGTGCCGGC
MetLeuArgArgAlaLeuLeuCysLeuAlaLeuThrValLeuPheArgAlaGlyAlaGly
-20                                        -10

GCCCCGACGAGGAGGACCACGTCCTGGTGCTCCATAAGGGCAACTTCGACGAGGCGCTG
AlaProAspGluGluAspHisValLeuValLeuHisLysGlyAsnPheAspGluAlaLeu
 1                                  10                      20

GCGGCCACAAGTACCTGCTGGTGGAGTTCTACGCCCCATGGTGCGGCCACTGCAAGGCT
AlaAlaHisLysTyrLeuLeuValGluPheTyrAlaProTrpCysGlyHisCysLysAla
                                30                          40

CTGGCCCCGGAGTATGCCAAAGCAGCTGGGAAGCTGAAGGCAGAAGGTTCTGAGATCAGA
LeuAlaProGluTyrAlaLysAlaAlaGlyLysLeuLysAlaGluGlySerGluIleArg
                                50                          60

Fig. 2-2

CTGGCCAAGGTGGATGCCACTGAAGAGTCTGACCTGGCCCAGCAGTATGGTGTCCGAGGC
LeuAlaLysValAspAlaThrGluGluSerAspLeuAlaGlnGlnTyrGlyValArgGly
70                                                        80

TACCCCACCATCAAGTTCTTCAAGAATGGAGACACAGCTTCCCCCAAAGAGTACACAGCT
TyrProThrIleLysPhePheLysAsnGlyAspThrAlaSerProLysGluTyrThrAla
90                                                       100

GGCCGAGAAGCGGATGATATCGTGAACTGGCTGAAGAAGCGCACGGGCCCCGCTGCCAGC
GlyArgGluAlaAspAspIleValAsnTrpLeuLysLysArgThrGlyProAlaAlaSer
110                                                      120

ACGCTGTCCGACGGGGCTGCTGCAGAGGCCTTGGTGGAGTCCAGTGAGGTGGCCGTCATT
ThrLeuSerAspGlyAlaAlaAlaGluAlaLeuValGluSerSerGluValAlaValIle
130                                                      140

GGCTTCTTCAAGGACATGGAGTCGGACTCCGCAAAGCAGTTCTTCTTGGCAGCAGAGGTC
GlyPhePheLysAspMetGluSerAspSerAlaLysGlnPheLeuLeuAlaAlaGluVal
150                                                      160

ATTGATGACATCCCCTTCGGGATCACATCTAACAGCGATGTGTTCTCCAAATACCAGCTG
IleAspAspIleProPheGlyIleThrSerAsnSerAspValPheSerLysTyrGlnLeu
170                                                      180

GACAAGGATGGGGTTGTCCTCTTTAAGAAGTTTGACGAAGGCCGGAACAACTTTGAGGGG
AspLysAspGlyValValLeuPheLysLysPheAspGluGlyArgAsnAsnPheGluGly
190                                                      200

GAGGTCACCAAAGAAAAGCTTCTGGACTTCATCAAGCACAACCAGTTGCCCCTGGTCATT
GluValThrLysGluLysLeuLeuAspPheIleLysHisAsnGlnLeuProLeuValIle
210                                                      220

0 277 563

Fig. 2-3

GAGTTCACCGAGCAGACAGCCCCGAAGATCTTCGGAGGGGAAATCAAGACTCACATCCTG
GluPheThrGluGlnThrAlaProLysIlePheGlyGlyGluIleLysThrHisIleLeu
230                                                       240

CTGTTCCTGCCGAAAAGCGTGTCTGACTATGAGGGCAAGCTGAGCAACTTCAAAAAAGCG
LeuPheLeuProLysSerValSerAspTyrGluGlyLysLeuSerAsnPheLysLysAla
250                                                       260

GCTGAGAGCTTCAAGGGCAAGATCCTGTTTATCTTCATCGACAGCGACCACACTGACAAC
AlaGluSerPheLysGlyLysIleLeuPheIlePheIleAspSerAspHisThrAspAsn
270                                                       280

CAGCGCATCCTGGAATTCTTCGGCCTAAAGAAAGAGGAGTGCCCGGCCGTGCGCCTCATC
GlnArgIleLeuGluPhePheGlyLeuLysLysGluGluCysProAlaValArgLeuIle
290                                                       300

ACGCTGGAGGAGGAGATGACCAAATATAAGCCAGAGTCAGATGAGCTGACGGCAGAGAAG
ThrLeuGluGluGluMetThrLysTyrLysProGluSerAspGluLeuThrAlaGluLys
310                                                       320

ATCACCGAGTTCTGCCACCGCTTCCTGGAGGGCAAGATTAAGCCCCACCTGATGAGCCAG
IleThrGluPheCysHisArgPheLeuGluGlyLysIleLysProHisLeuMetSerGln
330                                                       340

GAGCTGCCTGACGACTGGGACAAGCAGCCTGTCAAAGTGCTGGTTGGGAAGAACTTTGAA
GluLeuProAspAspTrpAspLysGlnProValLysValLeuValGlyLysAsnPheGlu
350                                                       360

GAGGTTGCTTTTGATGAGAAAAAGAACGTCTTTGTAGAGTTCTATGCCCCGTGGTGCGGT
GluValAlaPheAspGluLysLysAsnValPheValGluPheTyrAlaProTrpCysGly
370                                                       380

0 277 563

Fig. 2-4

CACTGCAAGCAGCTGGCCCCCATCTGGGATAAGCTGGGAGAGACGTACAAGGACCACGAG
HisCysLysGlnLeuAlaProIleTrpAspLysLeuGlyGluThrTyrLysAspHisGlu
390                                                        400

AACATAGTCATCGCCAAGATGGACTCCACGGCCAACGAGGTGGAGGCGGTGAAAGTGCAC
AsnIleValIleAlaLysMetAspSerThrAlaAsnGluValGluAlaValLysValHis
410                                                        420

AGCTTCCCCACGCTCAAGTTCTTCCCCGCCAGCGCCGACAGGACGGTCATCGACTACAAT
SerPheProThrLeuLysPhePheProAlaSerAlaAspArgThrValIleAspTyrAsn
430                                                        440

GGGGAGCGGACACTGGATGGTTTTAAGAAGTTCCTGGAGAGTGGTGGCCAGGATGGGGCC
GlyGluArgThrLeuAspGlyPheLysLysPheLeuGluSerGlyGlyGlnAspGlyAla
450                                                        460

GGAGATGATGACGATCTAGAAGATCTTGAAGAAGCAGAAGAGCCTGATCTGGAGGAAGAT
GlyAspAspAspAspLeuGluAspLeuGluGluAlaGluGluProAspLeuGluGluAsp
470                                                        480

GATGATCAAAAAGCTGTGAAAGATGAACTGTAACACAGAGAGCCAGACCTGGGCACCAAA
AspAspGlnLysAlaValLysAspGluLeu
490

CCCGGACCTCCCAGTGGGCTGCACACCCAGCAGCACAGCCTCCAGACGCCCGCAGACCCT

CCCAGCGAGGGAGCGTCGATTGGAAATGCAGGGAACTTTTCTGAAGCCACACTTCACTCT

ACCACACGTGCAAATCTAAACCGTCTTCCTTTGCTTTTCAACTTTTGGAAAAGGGTTTA

TTTCCAGGCCAGCCCAGCCCAGCCCATCTTGGTGGGCCTTTTTTTTTAAATCGTGATGTA

Fig. 2-5

CTTTTTTTGTACCTGGTTTTGTCCAGAGTGCTCGCTAAAATGTTTTGGACTCTCACGCTG

GCAATGTCTCTCATTCCTGTTAGGTTTATACTATCACTTTAAAAAAATTCCGTCTGTGGG

ATTTTTAGACATTTTTGGACGTCAGGGTGTGTGCTCCACCTTGGCCAGGCCTCCCTGGGA

CTCCTGCCCTCTGTGGGGCAGAACCAGGGAAGGCTGGACGGGTCCCTCACCTCATGCGGT

ATTGCCATGGTGGAGCGTGGCTCCTGCATCATTTGATTAAATGGAGACTTTCCGGTCTCT

GTCACAGGCCGCTCCCAACCGTGAGTGGAGGGTGTGGCTGGGCCAGGACAAGCCCAGCA

CTGTGCCAGGCAGAACCGGGACCCTTCGTTTCCAGGCTGGGAGACAGCCAAGGATGCTTG

GCCCCTCCTTCCCCAAGCCAGGGTCCTTATTGCTCTGTGATGTCCAGGGTGGCCTGAGG

AGCTGAATCACATGTTGACAGTTCTTCAGGCATTTCTACCACAATATTGGAATTGGACAC

ATTGGCCAAATAAAGTTAAAATTTTCTGCCAAAAAAAAAAA

0 277 563

Fig. 3-1

X-Ala-Pro-Asp-Glu-Glu-Asp-His-Val-Leu-Val-Leu-His-Lys-Gly-Asn-Phe-Asp-Glu-Ala-Leu

Ala-Ala-His-Lys-Tyr-Leu-Leu-Val-Glu-Phe-Tyr-Ala-Pro-Trp-Cys-Gly-His-Cys-Lys-Ala

Leu-Aka-Pro-Glu-Tyr-Ala-Lys-Ala-Ala-Gly-Lys-Leu-Lys-Ala-Glu-Gly-Ser-Glu-Ile-Arg

Leu-Ala-Lys-Val-Asp-Ala-Thr-Glu-Glu-Ser-Asp-Leu-Ala-Gln-Gln-Tyr-Gly-Val-Arg-Gly

Tyr-Pro-Thr-Ile-Lys-Phe-Phe-Lys-Asn-Gly-Asp-Thr-Ala-Ser-Pro-Lys-Glu-Tyr-Thr-Ala

Gly-Arg-Glu-Ala-Asp-Asp-Ile-Val-Asn-Trp-Leu-Lys-Lys-Arg-Thr-Gly-Pro-Ala-Ala-Ser

Thr-Leu-Ser-Asp-Gly-Ala-Ala-Ala-Glu-Ala-Leu-Val-Glu-Ser-Ser-Glu-Val-Ala-Val-Ile

Gly-phe-Phe-Lys-Asp-Met-Glu-Ser-Asp-Ser-Ala-Lys-Gln-Phe-Leu-Leu-Ala-Ala-Glu-Val

Ile-Asp-Asp-Ile-Pro-Phe-Gly-Ile-Thr-Ser-Asn-Ser-Asp-Val-Phe-Ser-Lys-Tyr-Gln-Leu

Asp-Lys-Asp-Gly-Val-Val-Leu-phe-Lys-Lys-Phe-Asp-Glu-Gly-Arg-Asn-Asn-Phe-Glu-Gly

Glu-Val-Thr-Lys-Glu-Lys-Leu-Leu-Asp-Phe-Ile-Lys-His-Asn-Gln-Leu-Pro-Leu-Val-Ile

Glu-Phe-Thr-Glu-Gln-Thr-Ala-Pro-Lys-Ile-Phe-Gly-Gly-Glu-Ile-Lys-Thr-His-Ile-Leu

Leu-Phe-Leu-Pro-Lys-Ser-Val-Ser-Asp-Tyr-Glu-Gly-Lys-Leu-Ser-Asn-Phe-Lys-Lys-Ala

0 277 563

Fig. 3-2

270
Ala-Glu-Ser-Phe-Lys-Gly-Lys-Ile-Leu-Phe-Ile-Phe-Ile-Asp-Ser-Asp-His-Thr-Asp-Asn
280

290
Gln-Arg-Ile-Leu-Glu-Phe-Phe-Gly-Leu-Lys-Lys-Glu-Glu-Cys-Pro-Ala-Val-Arg-Leu-Ile
300

310
Thr-Leu-Glu-Glu-Glu-Met-Thr-Lys-Tyr-Lys-Pro-Glu-Ser-Asp-Glu-Leu-Thr-Ala-Glu-Lys
320

330
Ile-Thr-Glu-Phe-Cys-His-Arg-Phe-Leu-Glu-Gly-Lys-Ile-Lys-Pro-His-Leu-Met-Ser-Gln
340

350
Glu-Leu-Pro-Asp-Asp-Trp-Asp-Lys-Gln-Pro-Val-Lys-Val-Leu-Val-Gly-Lys-Asn-Phe-Glu
360

370
Glu-Val-Ala-Phe-Asp-Glu-Lys-Lys-Asn-Val-Phe-Val-Glu-Phe-Tyr-Ala-Pro-Trp-Cys-Gly
380

390
His-Cys-Lys-Gln-Leu-Ala-Pro-Ile-Trp-Asp-Lys-Leu-Gly-Glu-Thr-Tyr-Lys-Asp-His-Glu
400

410
Asn-Ile-Val-Ile-Ala-Lys-Met-Asp-Ser-Thr-Ala-Asn-Glu-Val-Glu-Ala-Val-Lys-Val-His
420

430
Ser-Phe-Pro-Thr-Leu-Lys-Phe-Phe-Pro-Ala-Ser-Ala-Asp-Arg-Thr-Val-Ile-Asp-Tyr-Asn
440

450
Gly-Glu-Arg-Thr-Leu-Asp-Gly-Phe-Lys-Lys-Phe-Leu-Glu-ser-Gly-Gly-Gln-Asp-Gly-Ala
460

470
Gly-Asp-Asp-Asp-Asp-Leu-Glu-Asp-Leu-Glu-Glu-Ala-Glu-Glu-Pro-Asp-Leu-Glu-Glu-Asp
480

490
Asp-Asp-Gln-Lys-Ala-Val-Lys-Asp-Glu-Leu

X: H, Met or
Met-Leu-Arg-Arg-Ala-Leu-Leu-Cys-Leu-Ala-Leu-Thr-Val-Leu-Phe-Arg-Ala-Gly-Ala-Gly

0 277 563

# Fig. 4-1

```
                                           460          470          480
                              Y- GC  CCCCGACGAG  GAGGACCACG  TCCTGGTGCT

    490          500          510          520          530          540
CCATAAGGGC  AACTTCGACG  AGGCGCTGGC  GGCCCACAAG  TACCTGCTGG  TGGAGTTCTA

    550          560          570          580          590          600
CGCCCCATGG  TGCGGCCACT  GCAAGGCTCT  GGCCCCGGAG  TATGCCAAAG  CAGCTGGGAA

    610          620          630          640          650          660
GCTGAAGGCA  GAAGGTTCTG  AGATCAGACT  GGCCAAGGTG  GATGCCACTG  AAGAGTCTGA

    670          680          690          700          710          720
CCTGGCCCAG  CAGTATGGTG  TCCGAGGCTA  CCCCACCATC  AAGTTCTTCA  AGAATGGAGA

    730          740          750          760          770          780
CACAGCTTCC  CCCAAAGAGT  ACACAGCTGG  CCGAGAAGCG  GATGATATCG  TGAACTGGCT

    790          800          810          820          830          840
GAAGAAGCGC  ACGGGCCCCG  CTGCCAGCAC  GCTGTCCGAC  GGGGCTGCTG  CAGAGGCCTT

    850          860          870          880          890          900
GGTGGAGTCC  AGTGAGGTGG  CCGTCATTGG  CTTCTTCAAG  GACATGGAGT  CGGACTCCGC

    910          920          930          940          950          960
AAAGCAGTTC  TTGTTGGCAG  CAGAGGTCAT  TGATGACATC  CCCTTCGGGA  TCACATCTAA

    970          980          990         1000         1010         1020
CAGCGATGTG  TTCTCCAAAT  ACCAGCTGGA  CAAGGATGGG  GTTGTCCTCT  TTAAGAAGTT

   1030         1040         1050         1060         1070         1080
TGACGAAGGC  CGGAACAACT  TTGAGGGGGA  GGTCACCAAA  GAAAAGCTTC  TGGACTTCAT
```

0 277 563

Fig. 4-2

```
       1090           1100           1110           1120           1130           1140
CAAGCACAAC CAGTTGCCCC TGGTCATTGA GTTCACCGAG CAGACAGCCC CGAAGATCTT

       1150           1160           1170           1180           1190           1200
CGGAGGGGAA ATCAAGACTC ACATCCTGCT GTTCCTGCCG AAAAGCGTGT CTGACTATGA

       1210           1220           1230           1240           1250           1260
GGGCAAGCTG AGCAACTTCA AAAAGCGGC TGAGAGCTTC AAGGGCAAGA TCCTGTTTAT

       1270           1280           1290           1300           1310           1320
CTTCATCGAC AGCGACCACA CTGACAACCA GCGCATCCTG GAATTCTTCG GCCTAAAGAA

       1330           1340           1350           1360           1370           1380
AGAGGAGTGC CCGGCCGTGC GCCTCATCAC GCTGGAGGAG GAGATGACCA AATATAAGCC

       1390           1400           1410           1420           1430           1440
AGAGTCAGAT GAGCTGACGG CAGAGAAGAT CACCGAGTTC TGCCACCGCT TCCTGGAGGG

       1450           1460           1470           1480           1490           1500
CAAGATTAAG CCCCACCTGA TGAGCCAGGA GCTGCCTGAC GACTGGGACA AGCAGCCTGT

       1510           1520           1530           1540           1550           1560
CAAAGTGCTG GTTGGGAAGA ACTTTGAAGA GGTTGCTTTT GATGAGAAAA AGAACGTCTT

       1570           1580           1590           1600           1610           1620
TGTAGAGTTC TATGCCCGT GGTGCGGTCA CTGCAAGCAG CTGGCCCCA TCTGGGATAA

       1630           1640           1650           1660           1670           1680
GCTGGGAGAG ACGTACAAGG ACCACGAGAA CATAGTCATC GCCAAGATGG ACTCCACGGC

       1690           1700           1710           1720           1730           1740
CAACGAGGTG GAGGCGGTGA AAGTGCACAG CTTCCCCACG CTCAAGTTCT TCCCCGCCAG
```

0 277 563

Fig. 4-3

```
     1750          1760          1770          1780          1790          1800
CGCCGACAGG  ACGGTCATCG  ACTACAATGG  GGAGCGGACA  CTGGATGGTT  TTAAGAAGTT

     1810          1820          1830          1840          1850          1860
CCTGGAGAGT  GGTGGCCAGG  ATGGGCCGG  AGATGATGAC  GATCTAGAAG  ATCTTGAAGA

     1870          1880          1890          1900          1910
AGCAGAAGAG  CCTGATCTGG  AGGAAGATGA  TGATCAAAAA  GCTGTGAAAG  ATGAACTG
```

Y : ATG or

ATGCTGCGCCGCGCTCTGCTCTGCCTGGCCCTGACCGTCCTATTCCGCGCGGGTGCCGGC

0 277 563

Fig. 5

Fig. 6

| DOCUMENTS CONSIDERED TO BE RELEVANT | | | EP 88100923.7 |
|---|---|---|---|

| Category | Citation of document with indication, where appropriate, of relevant passages | Relevant to claim | CLASSIFICATION OF THE APPLICATION (Int Cl 4) |
|---|---|---|---|
| D,A | NATURE, vol. 317, no. 6032, September 19, 1985, New York, London<br><br>J.C. EDMAN et al.; "Sequence of protein disulphide isomerase and implications of its relationship to thioredoxin"<br>pages 267-270<br><br>* Totality *<br><br>-- | 1-3 | C 12 N 15/00<br>C 12 N 9/90<br>C 07 H 21/04 |
| D,A | THE BIOCHEMICAL JOURNAL, vol. 213, 1983, London<br><br>N. LAMBERT et al.; "Structural properties of homogeneous protein disulphide-isomerase from bovine liver purified by a rapid high-yielding procedure"<br>pages 225-234<br><br>* Totality *<br><br>-- | 1 | |
| A | THE BIOCHEMICAL JOURNAL, vol. 191, 1980, London<br><br>D.A. HILLSON et al.; "Bovine liver thiol-protein disulphide oxido-reductases"<br>pages 389-393<br><br>* Totality *<br><br>-- | 1 | TECHNICAL FIELDS SEARCHED (Int. Cl.4)<br><br>C 12 N<br>C 07 H |

The present search report has been drawn up for all claims

| Place of search | Date of completion of the search | Examiner |
|---|---|---|
| VIENNA | 05-05-1988 | WOLF |

| | DOCUMENTS CONSIDERED TO BE RELEVANT | | EP 88100923.7 |
|---|---|---|---|
| Category | Citation of document with indication, where appropriate, of relevant passages | Relevant to claim | CLASSIFICATION OF THE APPLICATION (Int. Cl.4) |
| D,A | THE BIOCHEMICAL JOURNAL, vol. 213, 1983, London<br><br>N. LAMBERT et al.; "Kinetics and specificity of homogeneous protein disulphide-isomerase in protein disulphide isomerization and in thiol-protein-disulphide oxido-reduction"<br>pages 235-243<br><br>* Totality * | 1 | |
| | -- | | |
| D,A | TRENDS IN BIOCHEMICAL SCIENCES, vol. 9, October 1984, Amsterdam<br><br>R.B. FREEMAN; "Native disulphide bond formation in protein bio-synthesis: evidence for the role of protein disulphide isomerase"<br>pages 438-441<br><br>* Totality * | 1 | TECHNICAL FIELDS SEARCHED (Int. Cl.4) |
| | -- | | |
| D,A | METHODS IN ENZYMOLOGY, vol. 107, part B, 1984, Orlando, USA<br><br>D.A. HILLSON et al.; "Formation and Isomerization of Disulphide Bonds in Proteins: Protein Disulphide-isomerase"<br>pages 281-294<br><br>* Totality * | 1 | |
| | ---- | | |

The present search report has been drawn up for all claims

| Place of search | Date of completion of the search | Examiner |
|---|---|---|
| VIENNA | 05-05-1988 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82